# EUROPEAN PATENT APPLICATION

(11) **EP 3 839 883 A1**
(43) Date of publication of application: **23.06.2021**
(21) Application number: 19217509.9
(22) Date of filing: 18.12.2019
(51) Int. Cl.: G06T 7/00, A61B 5/00, G01B 9/02, G06T 7/20

(54) **A METHOD AND A SYSTEM FOR OBTAINING IMAGE DATA OF AN OBJECT**

(71) Applicant: Svendsen, Morten Bo Søndergaard, 2800 Kongens Lyngby (DK)
(72) Inventor: Svendsen, Morten Bo Søndergaard, 2800 Kongens Lyngby (DK); Hansen, Jan Bertholdt, 2800 Kongens Lyngby (DK)
(74) Representative: Inspicos P/S

(57) **Abstract**

When imaging an object with a camera, a periodic relative movement may be determined between the object and the camera. Then, the camera may be controlled to acquire the image at a particular point along the periodic movement. Multiple images may be taken in multiple periods but at the same position so that registration is simple.

## Description

The present invention relates to the obtaining of image data of an object in the situation where a periodic relative movement is seen between the camera and the object.

Imaging elements may be seen in WO2017062759, US2014/276099, US2017/354392, US2017017858, CN107307848, CN204765619U, WO2014009859, WO2011117779, RU2573053, US2016270672, WO2010017508, CN102357033, "Correcting for motion artifact in handheld laser speckle images", Lertsakdadet B et al., Journal of Biomedical Optics, March 2018, page 036006 (7 pp.), NR 3 VOL 23., "Development of a handheld blood flow measurement system using laser speckle flowgraphy", Min-Chul Lee et al., Optical Society of Japan, co-published with Springer, April 2015, page 308-314, NR 2, VOL 22., "Handheld, point-of-care laser speckle imaging", Farraro R et al., Journal of Biomedical Optics, September 2016, page 094001 (6 pp.), NR 9 VOL 21., and "PulseCam: High-resolution blood perfusion imaging using a camera and a pulse oximeter", Kumar Mayank et al., 16 August 2016, 38th Annual International Conference of the IEEE Engineering in Medicine and Biology Society (EMBC), page 3904-3909.

In a first aspect, the invention relates to a method of obtaining image data of an object, the method comprising:
- determining a periodic movement between an imaging element and an imaged part of the object,
- subsequently controlling the imaging element to provide the imaging data at a predetermined position of the period of the periodic movement.

In the present context, image data may be data of the type acquired by a normal camera on a normal day.

Image data may be represented in an at least two-dimensional fashion.

In a preferred embodiment, image data may be data representing a physical surface or structure, such as a portion of a human or animal body, a structure or the like. Image data may represent the surface, such as a normal image. Alternatively, the image data may be data where a colour of the actual surface/object has been replaced or added information relating to an analysis performed on the basis of the image data, such as flow or relative movement one portion of the surface/structure vis-à-vis another portion of the surface/structure.

Typically, images are 2D representations of 2D or 3D elements, where the 2D representation is a projection of the surface of the elements on to a surface or image plane. Usually, an image has, at a position often called a pixel, a colour or grey tone of a corresponding position of the element. Image data in the present context, may, at a position, comprise data or information in addition to or instead of such colour/grey tone. Such data or information may represent a value, such as a relative movement between a portion of the element at the corresponding position and other elements. As will be seen below, a vast number of measurement types may be employed. In a particular example, flow velocity and/or direction may be represented in the image data and in a position of the image data corresponding to a position or portion in the element where a flow or relative movement is seen vis-à-vis other portions of the element.

The image data may be represented in any manner. A large number of image formats exist, such as JPEG, TIFF, GIF, BMP or the like. In general, the image data may represent a number of portions or pixels each preferably at least representing a coordinate or position in an at least 2D surface and a value corresponding to a result of an analysis of the image data.

The image data is acquired during a period of time. During the period of time, a sensing portion of the imaging device may be exposed to radiation, such as light. In older camera types, a shutter will define the period of time during which radiation is allowed to reach a sensor. In other types of sensor, the sensor is constantly exposed to radiation but has a storage erased before the period of time starts and has the storage emptied when the period of time stops, so that the contents of the storage relates only to radiation received during the period of time. The image data thus relates to an image acquired during a period of time.

The object may be any type of object. Often, objects have portions with different velocities, so that an analysis of such relative velocity or motion may be made. In one embodiment, an LSCI analysis may be made in the image. This analysis is sensitive to relative movement not only within the object but also between the object and the imaging element and the object as well as between a radiation source and the object and/or the imaging element.

The imaging element need not image all of the object. A part of the object may be imaged, such as a limb or a part thereof of a person.

In this context, an imaging element may be a camera, such as a video camera, still camera, colour camera, black/white camera or the like. The technology may be analogue or digital, full frame, half frame, scanning or the like. The imaging element may have any suitable optics, such as lenses, light guides, shutters or the like, to convey radiation from the object to a sensing portion, such as a radiation sensitive chip, of the imaging element.

The imaging element may be used for a number of purposes. In one situation, the imaging element is used in an analysis of the object. In yet another situation, the imaging element is used for providing images of the object. Naturally, the situations may be combined.

The movement between the imaging element and the imaged part of the object causes the imaged part of the object to move in relation to the imaging element. Movement usually poses challenges during image acquisition, and especially when the resulting imaging data is to be used in an analysis of other movement, such as movement internally in the object.

The movement is periodic. A periodic movement may continuously move along the same track or pattern, which is a non-damped periodic movement. A damped periodic movement will be periodic but may follow a successively changing path due to the damping. The damping may be due to friction, such as air friction or the like, which will affect the velocity of the movement. A periodic movement may be reciprocating so that the movement is back and forth along the same track or path. Also reciprocating movements may be damped. A periodic movement may be the moon's path around the Earth. A reciprocating movement may be the movement of a pendulum in a pendulum clock. A pendulum swinging freely in air would be damped due to the air causing friction against the movement of the pendulum creating a damped movement.

A periodic movement may be one-dimensional, two-dimensional or three-dimensional.

A periodic movement may be created by vibration in an arm holding the imaging element. Stiff elements naturally may vibrate due to their stiffness. Vibrations usually are damped oscillations.

A periodic movement may also be seen when the imaging element is attached to a wall/ceiling/floor which vibrates, such as due to a heavy vehicle passing or other vibrations of the structure.

Further movements may be seen when the imaging element is handheld by a person, where the breathing and/or pulse of the person may make the hand/arm of the person move slightly. This movement again is periodic, as the breathing and pulse are periodic.

Additionally, relative movement may be seen when the object or the part thereof moves periodically. Thus, if the object breathes or has a pulse, a periodic movement may be also seen.

A periodic movement may be detected or even assumed. That the movement is periodic may be ascertained if tracked over more than one period thereof. If the movement is assumed to be periodic, its path may be determined even without determining it over a full period.

The controlling of the imaging element may be achieved in a number of manners. In one manner, the controlling element may acquire images or provide imaging data only when instructed to do so. In that situation, the instruction may be forwarded to the imaging element to provide imaging information when in the predetermined position. The instruction then may be sent when the position of the movement is at or close to the predetermined position.

Alternatively, the movement may be predicted into the future and the instruction may be sent in advance with information of a time delay which should take place before the image information is provided or a point in time in the future could be forwarded at which the image information is to be obtained.

As mentioned above, the acquiring of an image or image information may be the opening of a shutter/mirror, the emptying of bins, deletion of data or the like, so that the image or image information may be obtained.

In other situations the imaging element may generate a sequence of images or image information, where the imaging may then be controlled so as to provide an image or image information when in or at the position. Alternatively, the image or image information provided when in the predetermined position may be selected and output from the imaging element, whereas other images or image information may be discarded or not used in the present context. Clearly such images or image information may be used for other purposes, such as to determine the periodic movement.

As will be described below, the predetermined position may be selected using different criteria. In one situation, the position may be a position where the velocity in the periodic movement is as low as possible. For a reciprocating movement, this would be an extreme position where the velocity is zero.

For other movements, a central position, such as the lowest position in a pendulum movement, may be chosen, as this will be visited even when the movement is damped.

In a preferred embodiment, the providing step comprises controlling the imaging element to provide imaging data during multiple periods of the periodic movement, where each image data is obtained at the predetermined position of the period.

Then, a number of images or image data is provided over time from the same relative position in spite of the relative movement. This has a number of advantages.

Clearly, monitoring the object may be desired over time, such as if the object is having surgery and it is desired to monitor the blood flow or the like in the object. In this manner, imaging of the object may be desired, as imaging may be used for illustrating or detecting relative movements within the object, such as blood vessels moving/pulsating.

One manner of obtaining this is to launch coherent radiation on to the object and detect smearing of intensity peaks thereon. This smearing is created by relative movement. Intensity peaks are well defined on stationary elements.

However, measurements of this type are very sensitive to movement between the object and the imaging element and between the light source and the object. Thus, by reducing or removing the effect of the relative movement between the imaging element and the object, a better determination may be performed.

If the radiation emitter is connected to, such as fixed to, the imaging element, another of the relative movements is addressed.

As mentioned, it may be desired in this context to provide the imaging data when the velocity of the periodic movement is as low as possible.

The method preferably further comprises the step of, during at least two of the multiple periods, launching radiation toward the imaged part of the object.

It may, in some situations, though, be desired that the launching step comprising launching radiation with different wavelengths toward the area during different periods. Thus, during one period, the radiation may be coherent radiation to facilitate the above measurement of relative velocities in the object. In another period, the radiation may be white light for the providing of a standard colour image from which the object may be seen. In a third period, radiation at a predetermined wavelength may be launched on to the object and radiation of the same or another wavelength may be sensed by the imaging element (such as using a filter) in order to obtain an absorption measurement or a fluorescence measurement.

In such situations, the method may further comprise the step of overlapping the image data provided during the multiple periods.

Overlapping of images of the same object often has the problem of registering - that the images are taken from different positions or the object was displaced between images, so that each image needs to be translated or even rotated in order for the images to correspond.

Overlapping images has the advantage that more information may be provided. If all images relate to the same information, such as an analysis, a result, or the like, adding images to the analysis or overlapping the images will add to the data and thus often increase the quality of the image or analysis. If, for example, the images relate to absorption in the object, multiple images will increase the quality of the absorption measurement. Also, if the image relates to relative movements in the object, adding more images may add to the quality of the movement determination.

On the other hand, if the images relate to different information, such as if the images relate to radiation of different wavelengths, images with different information may be combined. One image may be a standard colour image taken while illuminating the object with white light, where another image may relate to an analysis, such as illustrating absorption or relative movement over the image. Then, the absorption information may overlay the colour image so that absorption may be seen while the boundaries etc. of the object may be seen.

Again, the overlaying of the images is simple in that the images are taken from the same relative position or at least substantially the same relative position. No translation of one image relative to another image needs be performed.

An overlaying of the images may be a combination of the information of the images, such as in each portion or pixel. The combination may be a combination of the colour/intensities, for example. In this manner, boundaries and the like may still be visible in the colour image.

Such combination may be performed in a plurality of manners. In one manner, only the boundary of the object or relevant portions thereof, such as a wound or the like, may be retained, where the inner portions of the outline may be filled-in with information relating to the results of the analysis, such as the absorption/fluorescence/velocity.

Another aspect of the invention relates to an apparatus for providing image data of an object, the apparatus comprising:
- an imaging means configured to provide image data of an imaged part of the object,
- a sensor configured to determine a periodic movement between an imaging element and the imaged part of the object,
- a first processor configured to control the imaging element to provide the imaging data at a predetermined position of the period of the periodic movement.

In this relation, the imaging means, sensor and first processor may be attached to each other or separate elements which may be in communication with each other, such as via wires or wirelessly.

The image data, object, imaging element and the like may be as described above.

Naturally, all aspects, embodiments, situations and the like may be combined in any manner desirable.

As mentioned above, the imaging element may be a camera, and the imaging information may be an image represented in any desired manner.

The imaging information may subsequently be altered to represent information relating to the object. Often, the information in each portion of the imaging information will relate to a pertaining portion of the object or other elements imaged in the image. For example, if the image relates to the resulting intensity spots generated by coherent radiation launched on to the object, blurring of the spots will describe relative movement of the pertaining portions of the object. The image or imaging information output from the imaging element may then represent these intensity spots. The imaging information may then be altered to represent the movement in another manner, such as a colour, with vectors or numbers illustrating the amount of the velocity, and the like. This altered imaging information is more easily understood by a human operator.

Only a part of the object may be imaged. This part may be indicated on a display illustrating what is imaged, or an indication may be made on or toward the object indicating which part is imaged. This indication may be the launching of e.g. radiation toward the part or toward an outline or border thereof to indicate where the imaging element is directed.

The sensor may be any type of movement sensor. One sensor type may be an accelerometer, gyrometer or the like configured to sense its own motion relative to standstill. This may be relevant when the object is more or less stationary and where the relative movement primarily or completely is a movement of the imaging element.

Also, one or more distance sensors may be used. If it is assumed that the sensors are provided in a stationary setting, such as a room, distance sensors directed at floor/ceiling/walls will reveal movement of the sensors. Also, if a sensor is directed toward the object, relative motion of the object may also be determined. Distance sensors may be based on time-of-flight measurements based on radiation and/or sound, such as ultrasound. Other types of distance sensors exist.

Naturally, the sensor(s) may be attached to the imaging element to form a unit which may be easily handled. This is especially the case when the unit is handheld, and thus operable by an operator. Preferably, the imaging element has a handheld unit comprising the imaging device, or at least an imaging plane or the sensitive portion thereof. In this context, a unit may be handheld if it weighs no more than 2kg, such as no more than 1kg, such as no more than 750g, such as no more than 500g. Also, a handheld unit preferably has rather small dimensions, such as no more than 40cm along any dimension. The handheld unit may be connected to cables feeding signals/power to/from the unit. Otherwise, it may be battery operated and utilize wireless communication.

When the unit is handheld, it may have undesired movements in relation to the object, whereby it is desired to have the sensor(s) in the unit.

In another situation, the sensor(s) may be attached to the object. Then, one sensor may be facing the imaging element in order to again determine relative movement. Sensors on or at the object may especially be desired if the object may move, such as due to breathing and/or pulse. Also sensors may be desired on or at the object, if the object is provided on a support which may vibrate.

In yet another embodiment, one or more sensors may be provided which are not attached to neither the imaging element nor the object but to another element, such in relation to a predetermined structure, such as a wall, which sensor may determine relative movement between itself and the imaging element and/or in relation to itself and the object. Also in this manner, the relative movement may be determined.

Other types of movement sensors may be used, such as using a principle where coherent radiation is provided on the object. Relative movement between the object and the radiation emitter and/or the imaging element will create smearing of the intensity spots provided on the object. In addition, the positions of the spots in the imaging information may vary. From the position changes and/or the amount of smearing, the relative movement may be determined. It is noted that the coherent radiation may alternatively be fed on to the imaging element, if another imaging element is provided for this movement determination.

Alternatively, the radiation emitter may be attached to the object so that the radiation is fed on to the imaging element or a third element, such as a stationary element, where the relative movement may again be determined.

That the movement is periodic may be determined from data relating obtained from a number of the periods. A periodic movement may be one where the movement follows at least substantially the same path over and over again. In this respect, the movement may be seen as periodic if, in two subsequent periods, the movement covers a distance not varying more than 10%, such as 5%, such as 3% of the largest distance of the two periods. In addition or alternatively the movement may be seen as periodic, if the time duration of the period of one of the periods is between 90% and 110% of the time duration of the other period. Also, the movement may be periodic if the distance from each portion of one period is no farther from any portion of a neighbouring period than 10%, such as no farther than 7%, such as no farther than 5%, such as no farther than 3%, such as no farther than 1% of a total length of the path of the one period.

It is noted that the movement may be reciprocal. In this situation, the movement will more or less retrace the path, such as a pendulum in a pendulum clock. Thus, a reciprocal movement may have two points in time and two positions for each period where the velocity is zero and where the direction of the movement reverses. The time elapsed in each period may be determined between these positions. Also, the movement may be reciprocal if the distance from each portion of one period is no farther from any portion of a neighbouring period than 10%, such as no farther than 7%, such as no farther than 5%, such as no farther than 3%, such as no farther than 1% of a total length of the path of the one period. One exception may be close to the end portions, when the movement is damped, cf. below.

It is noted that the movement may be damped. Then, the periods will not be identical, but usually the velocity of the relative movement will slowly decrease. Then, the path of the movement may not be retraced but will alter from period to period.

If the movement was a circular or near-circular movement, or a 3D movement, such as on the surface of a sphere, the movement would describe a smaller and smaller diameter while the velocity along the periphery will reduce slowly. In this situation, the movement will not revert to a historic position.

In general, if the movement does not revert to the former position in the next period, the position may then be that closest to the former position. If the movement was near-circular, the nearest position would be that at the same angle, in a plane perpendicular to a central axis of the near-circular movement, around the axis.

If the movement is reciprocating, the end portions of successive periods will become increasingly closer to each other. The remainder of the movement may be along the same path.

A first processor is configured to control the imaging element to provide the imaging data at a predetermined position of the period of the periodic movement.

The predetermined position may, as described above, be any position along the path taken during the periodic movement. Depending on the movement and the reason for proving the imaging information, the position may be one frequented the most over time or one where the velocity is zero, for example.

In one embodiment, the first processor is configured to control the imaging element to provide imaging data during multiple periods of the periodic movement, usually consecutive periods, where each image data is obtained at the predetermined position of the period. As described above, this may facilitate combining the imaging data.

In one situation, it may be desired to provide the imaging data not only at the same position, but also when the movement is in the same direction. Imaging information is provided over a period of time, so it may be desired that the acquisition takes place over the same portion of the trajectory. Then, the sensor preferably is additionally configured to determine a direction of the movement.

Actually, it may be desired that the first processor is configured to control the imaging element to provide the image data during the multiple periods where each image data is obtained where the direction is the same in each of the multiple periods.

In one embodiment, the apparatus further comprises irradiating elements configured to launch, during at least two of the multiple periods, radiation toward the imaged part of the object. Above, it is described that the radiation may be white light enabling the providing of a colour image. The radiation may be of a particular wavelength absorbed by an interesting component of the object and/or which will cause this component to fluoresce.

Then, in one embodiment, the irradiating elements are configured to launch radiation with different wavelengths toward the area during different periods.

One embodiment further comprises a second processor for generating combined image data by overlapping the image data provided during the multiple periods. The first and second processors may be the same processor if desired. In this context, a processor may be any type of controller, processor, RISC chip, FPGA, DSP, software controllable or hardwired. Clearly, a combination of such elements may be used if desired.

In one embodiment, the second processor is configured to overlap the image data by combining the image data of the multiple periods into one image without translating one image data in relation to another. Thus, when the image data has the same number of e.g. pixels, the same pixels (same coordinate in the image) from different imaging data may simply be summed. Any algorithm may be used between the pixels of two or more imaging information to arrive at the pixel in the final imaging information.

In one embodiment, as described above, the sensor is configured to determine a reciprocating movement and wherein the first processor is configured to control the imaging element to provide the image data at an end of the reciprocating movement. The end of the movement has the lowest relative velocity. Naturally, the movement may be 2 or 3 dimensional so that the movement need not be zero at the end. A free hanging pendulum will move along the surface of a sphere and may have a movement which seen from above is elliptical. At the outer portions along the longitudinal axis, the movement is perpendicular to the longitudinal axis, is the smallest along the trajectory but is still non-zero.

In one embodiment, the sensor is configured to determine a damped reciprocating movement and wherein the first processor is configured to control the imaging element to provide the image data at a centre point of the reciprocating movement. The centre point may be that directly between the end points of each period and may be the position where the velocity is the highest. However, this point is included in each period which may be an advantage.

In one embodiment, the apparatus further comprises a source of coherent radiation configured to launch coherent radiation toward the imaged area of the object so as to provide speckles on the imaged area, the apparatus further comprising a third processor configured to detect smearing of the speckles caused by the relative movement and determine the relative movement based on the smearing.

As described above, this type of hardware may be used for the determination of the movement. The same hardware may also or alternatively be used for determining relative movements in the object. Naturally, a second imaging element may be used for sensing the radiation relating to the intensity spots, if the first imaging element is to be used for other purposes. It may, for example, be desired that the imaging element used for receiving the intensity spot radiation has a filter eliminating radiation of other wavelengths.

In the following, preferred embodiments will be described with reference to the drawing, wherein:
- Figure 1 illustrates an apparatus imaging an object,
- Figure 2 illustrates an image taken by the apparatus of figure 1,
- Figure 3 illustrates images taken by the apparatus of figure 1, and
- Figure 4 illustrates two reciprocal movements, a non-damped and a damped.

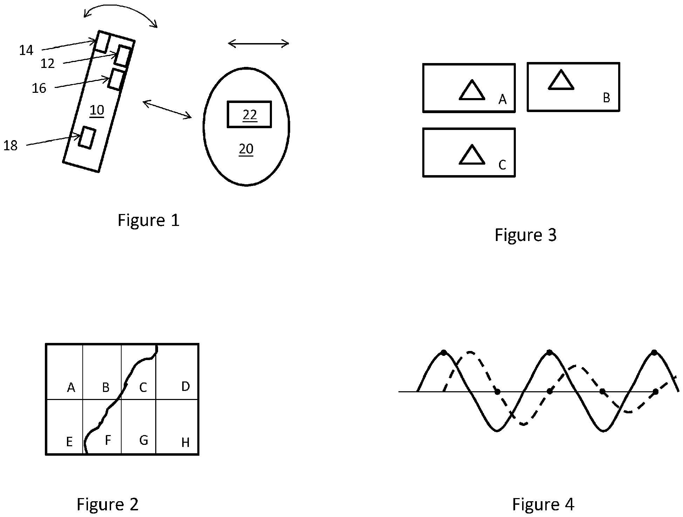

In figure 1, a handheld apparatus 10 comprises a camera 12 positioned and directed so as to provide images of an area 22 of an object 20. The object may be a patient and the apparatus 10 may be held by an operator during e.g. surgery of the patient where the imaged area 22 is the area of or an area of the surgery.

Relative movement may take place between the camera 12 and the area 22 for a number of reasons. The operator may be breathing, slightly displacing the camera 12 in relation to the area 22. The object or patient may also be breathing.

Also, a pulse and/or breathing of the operator and/or the patient/object may cause relative movement.

Naturally, the camera or apparatus may be held by a stationary object, such as a robot or an arm supported by, carried by or even fixed to a wall, ceiling or floor. This, however, does not take away the relative movement caused by the object. Also, an arm may itself vibrate and thus cause relative movement.

Relative movements may be in many directions and have many forms. A periodic movement may e.g. be along a circle or oval - or may be reciprocating so as to follow the same path back and forth. A few possible relative movements are indicated as reciprocating arrows in figure 1.

Breathing and pulse, however, as well as vibration, will create a periodic movement or even a reciprocating movement, which may be determined and even quantified.

To determine the relative movement, a sensor 14 is provided, such as in the apparatus. This sensor may operate in a number of manners. If the relative movement is caused by the apparatus 10 moving, the sensor may be an accelerometer sensing movement of the apparatus 10 and not taking into account any movement of the object 20.

Alternatively, the sensor may be a range finder positioned so as to determine, preferably in real time, a distance between the apparatus 10, such as the camera 12, and the object, such as the area 22. A range finder may be based on stereo imaging, lasers or the like. Combinations of sensors clearly may be used.

In one embodiment, the relative movement is determined by launching, from a radiation emitter 16, coherent radiation toward the area 22. Coherent radiation will create a pattern of intensity variation, often called speckles, on the area 22. Relative movement between the radiation source and the area 22 will cause this pattern to change. Obtaining image data over a period of time will have a resulting smearing of intensity peaks of this pattern. This is used for LSCI measurements, for example, for determining the movement of blood vessels in otherwise stationary tissue. This smearing may be used for determining relative movement between the camera and the area 22 and/or between the radiation emitter and the area 22. Thus, even if this pattern on the area 22 was stationary, such as if the radiation emitter was not a part of the handheld apparatus but stationary in relation to the area, relative movement between this pattern (the area) and a camera viewing the pattern will cause smearing and may thus be determined.

It is noted that the speckles may also smear due to internal movement in the area, such as when a blood vessel in the area pulses. However, such internal movement may be identified and not allowed to take part in the determination of the relative movement. The relative movement may be determined by averaging the movement determined in the area, potentially subtracting portions with deviating (as it is internal in the object) movement.

It is noted that the relative movement may cause a larger movement relative to some portions of the area than other portions of the area. Thus, individual portions of the area or of the image thereof may be identified and the relative movement may be determined in relation to each one. From the relative movements of individual portions, the overall relative movement may be determined. This overall relative movement may be a mean value of the determined relative movements, for example.

This is illustrated in figure 2, where the image is divided into 8 parts, A, B, C, D, E, F, G and H. Parts C and F, and to a minor degree B, illustrate an artery which may pulse and thus show internal movement relating to the surrounding tissue illustrated in the remaining parts of the image. This internal movement may be determined or identified and the parts C and F excluded in the termination of the relative movement. Alternatively the relevant portions of parts C and F may be excluded.

For the remaining parts, the relative movement seen may be that searched for, so that the smearing here may describe the relative movement between the camera and the object. The relative movement may be determined for each area. When these are not identical, the movement is not a translation direction toward or away from the area 22 or a rotation around an axis through the centre of the camera and the area. This may be identified and the relative movement detected, determined and quantified.

Thus, the use of the camera 12 or a separate camera of the apparatus 10 will enable the determination of relative movement between the apparatus 10 and the area 22.

Clearly, the sensor need not be provided in or attached to the apparatus. The relative movement may be determined in any manner.

Having determined the relative movement, information to that effect may be fed to a processor 18, which may be provided in the apparatus 10 or outside of the apparatus. The processor 18 receives the output of the sensor and determines one or more parameters of the periodic movement. Such parameters may be the period, a relative velocity and/or direction or the like.

The processor 18 then may control the camera 12 so that it takes an image of the area 22 at a predetermined relative position between the camera 12 and the area 22.

It may be desired that the image is taken when the relative velocity between the camera and the area is as low as possible. In a reciprocating movement this would be at either end of the reciprocating movement. In this manner, smearing of the image will be small, as any image is taken over a period of time during which the information on the sensor may move, causing smearing.

Also, knowledge of the periodic movement may be used to provide multiple images of the area where all images are taken when the relative position of the camera and the area is the same. In this manner, registering of the images is easy, as no registering is required.

This may be seen in figure 4 illustrating two reciprocal movements, a non-damped and a damped (hatched line). If the movement is determined to be or assumed to be non-damped, the point along the periodic movement where the image may be taken may be selected more freely. In the non-damped oscillation, the black dots illustrate positions where the velocity is low.

If the movement is assumed or determined to be damped, it may be decided to take the image around the centre (black dots) of the movement, as this position will be seen also during the dampened movement.

In figure 2, three images A, B and C are seen taken by the camera 12 of the area 22, which in this example has an element shaped as a triangle. Clearly, images A and C are identical, whereas the triangle in image B is in a different position.

The difference between images A and B may be caused by different relative positions existing when the images were taken.

Images A and C, however, are taken in different periods of the periodic movement but where the relative positions are the same. Thus, the images are identical.

This has the advantage that multiple images may be provided and added on top of each other, such as to generate a better statistic for e.g. quantification based on the images.

In another situation, the images are taken while different types of radiation are launched toward the area 22. Thus, an image where coherent radiation is launched toward the area may be combined with an image taken when white light is launched toward the area. The white light clearly makes it easy for an operator to orient him/herself in the image relative to the area 22 of the object. The coherent radiation may cause, as mentioned above, speckles which may be used for illustrating relative movement in the object itself (such as the movement of arteries during blood pumping).

The combination of such images still is simple, as the relative positioning is the same, so that the images may simply be added to each other without having to determine a relative position and a translation/rotation of one image in relation to another image.

## Claims

1. A method of obtaining image data of an object, the method comprising:
- determining a periodic movement between an imaging element and an imaged part of the object,
- subsequently controlling the imaging element to provide the imaging data at a predetermined position of the period of the periodic movement.

2. A method according to claim 1, wherein the controlling step comprises controlling the imaging element to provide imaging data during multiple periods of the periodic movement, where each image data is obtained at the predetermined position of the period.

3. A method according to claim 2, wherein the determining step comprises additionally determining a direction of the movement.

4. A method according to claims 2 and 3, wherein the controlling step comprises controlling the imaging element to provide the image data during the multiple periods where each image data is obtained where the direction is the same in each of the multiple periods.

5. A method according to any of claims 2-4, the method further comprising the step of, during at least two of the multiple periods, launching radiation toward the imaged part of the object.

6. A method according to claim 5, wherein the launching step comprising launching radiation with different wavelengths toward the area during different periods.

7. A method according to any of claims 2-6, further comprising the step of overlapping the image data provided during the multiple periods.

8. A method according to claim 7, wherein the overlapping is the combination of the image data of the multiple periods into one image without translating one image data in relation to another.

9. A method according to any of the preceding claims, wherein the determining step comprises determining a reciprocating movement and wherein the controlling step comprises controlling the imaging element to provide the image data at an end of the reciprocating movement.

10. A method according to any of the preceding claims, wherein the determining step comprises determining a damped reciprocating movement and wherein the controlling step comprises controlling the imaging element to provide the image data at a centre point of the reciprocating movement.

11. A method according to any of the preceding claims, wherein the determining step comprises the steps of:
- launching coherent radiation toward the imaged area of the object so as to provide speckles on the imaged area,
- detecting smearing of the speckles caused by the relative movement and
- determining the relative movement based on the smearing.

12. An apparatus for providing image data of an object, the apparatus comprising:
- an imaging means configured to provide image data of an imaged part of the object,
- a sensor configured to determine a periodic movement between an imaging element and the imaged part of the object,
- a first processor configured to control the imaging element to provide the imaging data at a predetermined position of the period of the periodic movement.

13. An apparatus according to claim 12, wherein the first processor is configured to control the imaging element to provide imaging data during multiple periods of the periodic movement, where each image data is obtained at the predetermined position of the period.

14. An apparatus according to claim 12 or 13, wherein the sensor is additionally configured to determine a direction of the movement.

15. An apparatus according to any of claims 12-14, wherein the first processor is configured to control the imaging element to provide the image data during the multiple periods where each image data is obtained where the direction is the same in each of the multiple periods.

## Amended claims

### Amended claims in accordance with Rule 137(2) EPC.

1. A method of obtaining image data of an object, the method comprising:
- determining, using one or more sensors, a periodic movement between an imaging element, attached to the sensor(s), and an imaged part of the object,
- subsequently controlling the imaging element to provide the imaging data at a predetermined position of the period of the periodic movement.

2. A method according to claim 1, wherein the controlling step comprises controlling the imaging element to provide imaging data during multiple periods of the periodic movement, where each image data is obtained at the predetermined position of the period.

3. A method according to claim 2, wherein the determining step comprises additionally determining a direction of the movement.

4. A method according to claims 2 and 3, wherein the controlling step comprises controlling the imaging element to provide the image data during the multiple periods where each image data is obtained where the direction is the same in each of the multiple periods.

5. A method according to any of claims 2-4, the method further comprising the step of, during at least two of the multiple periods, launching radiation toward the imaged part of the object.

6. A method according to any of the preceding claims, wherein the one or more sensors comprises a movement sensor, such as an accelerometer or a gyrometer

7. A method according to any of the preceding claims, wherein the determining step comprises determining a reciprocating movement and wherein the controlling step comprises controlling the imaging element to provide the image data at an end of the reciprocating movement.

8. A method according to any of the preceding claims, wherein the determining step comprises determining a damped reciprocating movement and wherein the controlling step comprises controlling the imaging element to provide the image data at a centre point of the reciprocating movement.

9. A method according to any of the preceding claims, wherein the determining step comprises the steps of:
- launching coherent radiation toward the imaged area of the object so as to provide speckles on the imaged area,
- detecting smearing of the speckles caused by the relative movement and
- determining the relative movement based on the smearing.

10. An apparatus (10) for providing image data of an object (20), the apparatus comprising:
- an imaging means (12) configured to provide image data of an imaged part (22) of the object,
- a sensor (14) attached to the imaging means and configured to determine a periodic movement between an imaging element and the imaged part of the object,
- a first processor (18) configured to control the imaging element to provide the imaging data at a predetermined position of the period of the periodic movement.

11. An apparatus according to claim 10, wherein the first processor is configured to control the imaging element to provide imaging data during multiple periods of the periodic movement, where each image data is obtained at the predetermined position of the period.

12. An apparatus according to claim 10 or 11, wherein the sensor is additionally configured to determine a direction of the movement.

13. An apparatus according to any of claims 10-12, wherein the first processor is configured to control the imaging element to provide the image data during the multiple periods where each image data is obtained where the direction is the same in each of the multiple periods.

14. An apparatus according to any of claims 10-13, wherein the sensor comprises a movement sensor.

15. An apparatus according to any of claims 10-14, wherein the sensor comprises an accelerometer or a gyrometer.
